Europäisches Patentamt

European Patent Office

Office européen des brevets

(11) Publication number: **0 319 963 B1**

# EUROPEAN PATENT SPECIFICATION

(45) Date of publication of patent specification: **25.03.92** (51) Int. Cl.⁵: **A61K  47/00**, A61K 31/355, A61K 31/56

(21) Application number: **88120479.6**

(22) Date of filing: **07.12.88**

(54) **Preparations of vitamin E for external use.**

(30) Priority: **08.12.87 JP 310481/87**

(43) Date of publication of application:
**14.06.89 Bulletin  89/24**

(45) Publication of the grant of the patent:
**25.03.92 Bulletin  92/13**

(84) Designated Contracting States:
**AT BE CH DE ES FR GB GR IT LI LU NL SE**

(56) References cited:

CHEMICAL ABSTRACTS, vol. 104, no. 24, 16th
June 1986, page 360, abstract no. 213037v,
Columbus, Ohio, US; & JP-A-61 36 210 (LION
CORP.) 20-02-1986

CHEMICAL ABSTRACTS, vol. 105, no. 26, 29th
December 1986, page 365, abstract no.
232468y, Columbus, Ohio, US; & JP-A-61 176
535 (MORISHITA JINTAN CO., LTD)
08-08-1986

(73) Proprietor: **SHIONOGI & CO., LTD.**
**1-8, Doshomachi 3-chome Chuo-ku**
**Osaka 541(JP)**

(72) Inventor: **Saitoh, Izumi**
**1-2-23-601, Kamikoshien**
**Nishinomiya-shi Hyogo(JP)**
Inventor: **Kido, Shigeru**
**327-20, Mishima**
**Higashiosaka-shi Osaka(JP)**
Inventor: **Doi, Yoshio**
**10-10, Wakazono-cho**
**Ibaraki-shi Osaka(JP)**
Inventor: **Egawa, Shohei**
**2-4-15, Nanatsumatsu-cho**
**Amagasaki-shi Hyogo(JP)**

(74) Representative: **Vossius & Partner**
**Siebertstrasse 4 P.O. Box 86 07 67**
**W-8000 München 86(DE)**

## Description

This invention relates to preparations of vitamin-E for external use, which are designed for the protection of the skin.

Hitherto, many kinds of creams or lotions intended for housewives have been sold on the market whose recommended uses are the prevention of rough skin of the hand, chaps, cracks and frostbite. It is, however, generally believed that rough skin, chaps, cracks in the skin, cannot be prevented unless the sufferer stops doing kitchen work. The reason is that, in most cases the base ingredients used in conventional creams and lotions are oils. Therefore, these preparations are capable of repelling water for a short while after application, but come off easily. This is the reason for their short action on the skin. Another consequence is that an oily film or odor may be imparted to the tableware. Therefore, these preparations have been applied after meals or kitchen work, i.e. mainly at bedtime. Thus, a sufficient skin-protecting effect was difficult to obtain.

The present invention provides preparations for external use, comprising essentially 0.1% to 5% of tocopherol acetate (hereinafter referred to as vitamin E or simply as VE) and/or 0.05% to 0.3% of glycyrrhetinic acid, 1% to 15% of a methacrylic acid-ethyl acrylate copolymer, and 0.2% to 3% of a thickening agent and an alcoholic solvent. Percentages or ratios used in this invention are in weight (W/W).

In view of the problems stated above, the present inventors tried to develop a VE preparation which satisfies the following requirements:

a. Causing no strange feeling when applied. In other words, it must be capable of forming a flexible, thin, and very soft film.

b. Forming a strong film. In other words, it must form a film which is not washed off by water or neutral detergents for a long period of time and is not peeled off.

c. Causing no irritation.

The present invention can be achieved by dissolving, in an alcoholic solvent, 0.1 - 5% of VE, 0.05 - 0.3% of glycyrrhetinic acid (GRA), 0.2 - 3% of a cellulose derivative, and 1 - 15% of a copolymer of methacrylic acid and ethyl acrylate (MAA-EA).

The tocopherol acetate used in this invention is also known as vitamin E, which has a vasodilating action. Given this action, VE can be expected to effectively accelerate the healing of the damaged skin by improving the blood circulation or the flexibility of capillary vessels.

Enteric-soluble tocopherol preparations and oral compositions containing tocopherol or its esters are disclosed in JP-A-61 176, 535 and JP-A-61 36,210

In addition, GRA is expected to relieve inflammation at the damaged part of the skin and to protect the skin from inflammation because of its anti-inflammatory action. In this invention, other pharmaceutically active ingredients such as various vitamins or allantoin may be further contained.

MAA-EA means an acrylic copolymer in which the monomer ratio of ethyl acrylate to methacrylic acid is within a range of from 75/25 to 95/5, the residual monomers being 50 ppm or less and with practically no surfactants. The processes for preparing said copolymer are disclosed in JPN Pat. Appln. Nos. 61-253071 and 61-253072, the equivalent of which is EP-A-265228.

The alcoholic solvent in this invention is a lower alkanol including ethanol, propanol, and isopropanol, The alcohol may contain water, but it is preferable to use an alcohol mixture whose alcohol content is 60% or more. Alcohol mixtures whose alcohol content is below this value are not preferred for this invention, in consideration of the solubility of a thickening agent therein.

Cellulose derivatives such as ethyl cellulose (EC), hydroxypropyl cellulose (HPC), and hydroxypropylmethyl cellulose (HPMC), are preferably used as a thickening agent. Plasticizers, e.g., propylene glycol may be further added.

VE preparations for external use of the present invention form a film which causes no strange feeling when applied to the hands and additionally, the active ingredients therein act on the skin for a long period of time. The film formed on the skin does not come off during kitchen work, and besides, it never imparts odor or an oily film to meals or kitchenware.

Conventional creams and lotions have to be applied very often because their active ingredients are washed off whenever kitchen work is done. Moreover, they should even be capable of being washed off before kitchen work or knitting work so as not to impart odor or an oily film. Thus in the case of conventional preparations, the period in which the active ingredients act on the skin is very short in spite of frequent applications. This has made it difficult to cure rough skin, cracks in the skin, and frostbites.

The protective film formed by the preparation of this invention shows a strong resistance to water, neutral detergents, various oils and the like without preventing the skin from breathing. The preparations are very economical since the drug is effective for a long time whether applied once or several times a day.

Besides, they are easy to handle, since the film formed can be easily and completely washed off with soap or alcohol.

This invention is explained in more detail by the following Examples and Experiments,

Reference Example (Preparation of the Copolymer)

A closed type reaction vessel with a stirrer was purged with nitrogen and then charged with 236.1 parts by weight of deionized water. After adjusting the temperature in the reaction vessel to 80°C, 1.2 parts by weight of ammonium persulfate were added and the following monomer mixture was then added within 8 hours.

| EA | 85 parts by weight |
| MAA | 15 parts by weight |

Stirring was continued for another 8 hours while keeping the temperature in the reaction vessel at 80°C to complete the reaction. The solid content of the emulsion was 30%, in which EA was present in 37ppm, and MAA was present in less than 10ppm according to the residual monomer analysis by means of gas chromatography. The mean molecular weight of this copolymer was about 840,000.

EXAMPLE 1

Purified water (28.0g) was put in a closed type vessel equipped with a stirrer. The emulsion(5.0g as MAA-EA content) prepared in the Reference Example above and 1.0g of EC were gradually added thereto and the mixture was stirred until the copolymer was dissolved completely.

Isopropanol (65.3g) was added to the solution of the copolymer and the mixture was stirred to give a clear solution. In the clear solution thus obtained, 0.5g of VE and 0.2g of GRA were dissolved under stirring to give a VE preparation for external use.

EXAMPLEs 2 - 6

The following VE preparations were prepared in substantially the same manner as in Example 1.

Table 1

| Example | 2 | 3 | 4 | 5 | 6 |
|---|---|---|---|---|---|
| Component | (%) | (%) | (%) | (%) | (%) |
| MAA・EA | 1.0 | 10.0 | 15.0 | 5.0 | 5.0 |
| VE | 0.1 | 1.0 | 1.5 | 0.5 | 0.5 |
| GRA | 0.04 | 0.2 | 0.2 | 0.1 | 0.15 |
| EC | 0.2 | 2.0 | 3.0 | 1.0 | 1.0 |
| Purified water | 28.0 | 28.0 | 28.0 | 28.0 | 28.0 |
| Ethanol | Add to make 100 | | Add to make 100 | | |
| IP | | Add to make 100 | | Add to make 100 | Add to make 100 |
| (Remark) IP stands for Isopropanol. | | | | | |

EXPERIMENT 1

The water resistance of each film was studied in the following manner.

(Preparations for Study)

3

Preparation of Example 1 (This Invention: VE content 0.5%)

[ _Sahne® Cream (Eisai Co., Ltd)_

Commercially Available Cream        VE content 0.5%)

(Method)

1. Purified water (500ml) is put in a cylindrical vessel (inside diameter 15 cm, height 15 cm).

2. The preparation (0.5g each) containing VE is applied in the middle of the back of the left hand of male volunteers (n = 5) and is spread evenly and circularly to cover an area of 50 mm in diameter.

3. The left hand is dipped in the vessel described in Item (1) and is rubbed with a finger for 3 minutes to remove the formed film.

4. The water in the vessel (3) is collected, and the VE is extracted therefrom with chloroform.

5. The extract is evaporated in vacuo to give a residue, which is dissolved in tetrahydrofuran. The amount of VE removed from the application area is measured by HPLC.

6. The amount of VE still remaining on the application area is determined from the data in Item (5), from which the remaining rate of VE is calculated.

(Result)

Table 2

|  | Ratio of remaining VE (%) |
|---|---|
| This Invention | 98.2±2.0 |
| Sahne® Cream (Eisai Co., Ltd) | 10.7±4.3 |

The above results confirm that the preparations of this invention are much better than commercially available creams in their water-resistant property and in the percentage of active ingredient remaining after hand washings.

EXPERIMENT 2

The amount of active ingredient rubbed off by the contact with clothing was determined in the following manner.

(Preparations for Study)

Preparation of Example 1 (This Invention: VE content 0.5%)

[ _Sahne® Cream (Eisai Co., Ltd)_

Commercially Available Cream        VE content 0.5%)

(Method)

1. Preparation (0.5g each) is applied in the middle of the back of the hand and then spread evenly and circularly so as to cover an area of 50 mm in diameter.

2. Fifteen minutes after the application, the application area is softly wiped with a piece of cellulose nonwoven fabric.

3. The piece of fabric to which the test sample is adhered is subjected to extraction with tetrahydrofuran. The VE is quantitatively measured by HPLC.

4. Based on the amount of the VE in Item (3), the rate at which VE is washed off is calculated.

4

EP 0 319 963 B1

(Result)

Table 3

| | Come-off Rate of VE (%) |
|---|---|
| This Invention | 2.3 |
| Sahne® Cream Eisai Co., Ltd | 75.7 |

The above results confirm that when the preparations of this invention are applied, the amounts of active ingredient lost by the contact with clothing are much smaller than in the case of the commercially available creams.

**Claims**
**Claims for the following Contracting States : AT, BE, CH, DE, IT, LI, LU, NL, SE**

1.  A preparation for external use which comprises essentially 0.1% to 5% of tocopherol acetate and/or 0.05% to 0.3% of glycyrrhetinic acid, 1% to 15% of a methacrylic acid-ethyl acrylate copolymer, and 0.2% to 3% of a thickening agent and an alcoholic solvent.

2.  The preparation claimed in Claim 1, wherein said methacrylic acid-ethyl acrylate copolymer is an acrylic copolymer in which the monomer ratio of ethyl acrylate to methacrylic acid is within a range of from 75/25 to 95/5, with the residual monomers being 50 ppm or less and with practically no surfactants.

3.  The preparation claimed in Claim 1, wherein said thickening agent is ethyl cellulose, hydroxypropyl cellulose, or hydroxypropylmethyl cellulose.

4.  The preparation claimed in Claim 1, wherein said alcoholic solvent is ethanol or isopropanol which may contain water.

5.  The preparation of any of claims 1 to 4 being a pharmaceutical preparation.

**Claims for the following Contracting States : ES, GR**

1.  A preparation for external use which comprises essentially 0.1% to 5% of tocopherol acetate and/or 0.05% to 0.3% of glycyrrhetinic acid, 1% to 15% of a methacrylic acid-ethyl acrylate copolymer, and 0.2% to 3% of a thickening agent and an alcoholic solvent.

2.  The preparation claimed in Claim 1, wherein said methacrylic acid-ethyl acrylate copolymer is an acrylic copolymer in which the monomer ratio of ethyl acrylate to methacrylic acid is within a range of from 75/25 to 95/5, with the residual monomers being 50 ppm or less and with practically no surfactants.

3.  The preparation claimed in Claim 1, wherein said thickening agent is ethyl cellulose, hydroxypropyl cellulose, or hydroxypropylmethyl cellulose.

4.  The preparation claimed in Claim 1, wherein said alcoholic solvent is ethanol or isopropanol which may contain water.

**Revendications**
**Revendications pour les Etats contractants suivants : AT, BE, CH, DE, FR, GB, IT, LI, LU, NL, SE**

1.  Préparation à usage externe, qui comprend essentiellement 0,1 % à 5 % d'acétate de tocophérol et/ou 0,05 % à 0,3 % d'acide glycyrrhétinique, 1 % à 15 % d'un copolymère d'acide méthacrylique-acrylate d'éthyle et 0,2 % à 3 % d'un agent épaississant, et un solvant alcoolique.

5

**2.** Préparation suivant la revendication 1, caractérisée en ce que le copolymère d'acide méthacrylique-acrylate d'éthyle est un copolymère acrylique dans lequel le rapport des monomères de l'acrylate d'éthyle à l'acide méthacrylique se situe dans un intervalle de 75/25 à 95/5, les monomères résiduels constituant 50 ppm ou moins et avec pratiquement pas d'agents tensio-actifs.

**3.** Préparation suivant la revendication 1, caractérisée en ce que l'agent épaississant est de l'éthyl cellulose, de l'hydroxypropyl cellulose ou de l'hydroxypropylméthyl cellulose.

**4.** Préparation suivant la revendication 1, caractérisée en ce que le solvant alcoolique est de l'éthanol ou de l'isopropanol qui peut contenir de l'eau.

**5.** Préparation suivant l'une quelconque des revendications 1 à 4, constituée par une préparation pharmaceutique.

**Revendications pour les Etats contractants suivants : ES, GR**

**1.** Préparation à usage externe, qui comprend essentiellement 0,1 % à 5 % d'acétate de tocophérol et/ou 0,05 % à 0,3 % d'acide glycyrrhétinique, 1 % à 15 % d'un copolymère d'acide méthacrylique-acrylate d'éthyle et 0,2 % a 3 % d'un agent épaississant, et un solvant alcoolique.

**2.** Préparation suivant la revendication 1, caractérisée en ce que le copolymère d'acide méthacrylique-acrylate d'éthyle est un copolymère acrylique dans lequel le rapport des monomères de l'acrylate d'éthyle à l'acide méthacrylique se situe dans un intervalle de 75/25 à 95/5, les monomères résiduels constituant 50 ppm ou moins et avec pratiquement pas d'agents tensio-actifs.

**3.** Préparation suivant la revendication 1, caractérisée en ce que l'agent épaississant est de l'éthyl cellulose, de l'hydroxypropyl cellulose ou de l'hydroxypropylméthyl cellulose.

**4.** Préparation suivant la revendication 1, caractérisée en ce que le solvant alcoolique est de l'éthanol ou de l'isopropanol qui peut contenir de l'eau.

**Patentansprüche**
**Patentansprüche für folgende Vertragsstaaten : AT, BE, CH, DE, IT, LI, LU, NL, SE**

**1.** Präparat zur äußerlichen Anwendung, das im wesentlichen 0,1 bis 5% Tocopherolacetat und/oder 0,05 bis 0,3% Glycyrrhetinsäure, 1 bis 15% Methacrylsäure-Ethylacrylat-Copolymer und 0,2 bis 3% eines Verdickungsmittels und ein alkoholisches Lösungsmittel umfaßt.

**2.** Präparat nach Anspruch 1, wobei das Methacrylsäure-Ethylacrylat-Copolymer ein Acryl-Copolymer ist, in dem das Monomer-Verhältnis von Ethylacrylat zu Methacrylsäure im Bereich von 75/25 bis 95/5 liegt, wobei die verbliebenen Monomere 50 ppm oder weniger betragen und praktisch keine grenzflächenaktiven Stoffe vorliegen.

**3.** Präparat nach Anspruch 1, wobei das Verdickungsmittel Ethylcellulose, Hydroxypropylcellulose oder Hydroxypropylmethylcellulose ist.

**4.** Präparat nach Anspruch 1, wobei das alkoholische Lösungsmittel Ethanol oder Isopropanol, die Wasser enthalten können, ist.

**5.** Präparat nach einem der Ansprüche 1 bis 4, das ein Arzneimittel darstellt.

**Patentansprüche für folgende Vertragsstaaten : ES, GR**

**1.** Präparat zur äußerlichen Anwendung, das im wesentlichen 0,1 bis 5% Tocopherolacetat und/oder 0,05 bis 0,3% Glycyrrhetinsäure, 1 bis 15% Methacrylsäure-EthylacrylatCopolymer und 0,2 bis 3% eines Verdickungsmittels und ein alkoholisches Lösungsmittel umfaßt.

**2.** Präparat nach Anspruch 1, wobei das Methacrylsäure-Ethylacrylat-Copolymer ein Acryl-Copolymer ist, in dem das Monomer-Verhältnis von Ethylacrylat zu Methacrylsäure im Bereich von 75/25 bis 95/5 liegt, wobei die verbliebenen Monomere 50 ppm oder weniger betragen und praktisch keine grenzflächenaktiven Stoffe vorliegen.

3. Präparat nach Anspruch 1, wobei das Verdickungsmittel Ethylcellulose, Hydroxypropylcellulose oder Hydroxypropylmethylcellulose ist.

4. Präparat nach Anspruch 1, wobei das alkoholische Lösungsmittel Ethanol oder Isopropanol, die Wasser enthalten können, ist.